# EUROPEAN PATENT APPLICATION

(11) **EP 2 353 501 A1**
(43) Date of publication of application: **10.08.2011**
(21) Application number: 10153050.9
(22) Date of filing: 09.02.2010
(51) Int. Cl.: A61B 5/0215, A61M 25/10, A61M 1/10, A61M 5/142

(54) **Computer Controlled Post-Conditioning Device**

(71) Applicant: Sanofi-Aventis Deutschland GmbH, 65929 Frankfurt (DE)
(72) Inventor: Gille, Andreas, 3032 Ascot Vale, Victoria (AU); Schäfer. Matthias, 65926 Frankfurt am Main (DE)

(57) **Abstract**

The present invention provides a medical device adapted for use in post conditioning treatment comprising: a balloon catheter (1) functionally connected to at least one controllable automatically driven pump (4), and a control unit (5) for controlling the pump depending on at least one physiological parameter and/or catheter-specific parameter and/or in correspondence to a predefined, time-dependent algorithm according to a pre-established post conditioning perfusion protocol.

## Description

The present invention relates to a medical device adapted for use in post conditioning treatment. In particular, the present invention relates to a computer controlled post conditioning device.

Myocardial infarction (MI) or acute myocardial infarction (AMI), commonly known as a heart attack is the interruption of blood supply to part of the heart, commonly due to occlusion of a coronary artery. Percutaneous transluminal angioplasty, particularly coronary angioplasty using balloon catheters, has been developed as an alternative to bypass surgery for revascularization of stenotic and occluded body vessels, such as coronary arteries. In the standard angioplasty procedure the balloon catheter is positioned in the affected vessel so that by inflating the balloon multiple times the desired dilation of the occluded segment can be achieved. After the desired result of reopening the vessel has been obtained by the balloon inflations the balloon is completely deflated and the balloon catheter is withdrawn. Prompt restoration of blood flow to ischemic tissue limits infarct size. But the process of restoring the blood flow to the ischemic tissue, especially the myocardium, however, can also induce injury. This phenomenon, termed reperfusion injury, can paradoxically reduce the beneficial effects of the reperfusion. Reperfusion can also result in additional damage and complications. The reperfusion injury results from several complex and interdependent mechanisms and is distinct from the damage caused by ischemia per se. Those mechanisms involve endothelial cell dysfunction, micro vascular injury, alterations in intracellular Ca2+ handling, production of reactive oxygen species, changes in myocardial metabolism, and activation of neutrophils, platelets, and the complement system. Post conditioning treatment in the early stage of reperfusion (< 15 min) is known to reduce reperfusion injury. The known post conditioning treatment includes sequential/alternately manually controlled complete dilation and complete deflation of the balloon in the affected body lumen. Also between the phases of dilatation for re-opening the stenotic, occluded segment, already reperfusion occurs. Common dilatation devices are not especially designed for controlling the reperfusion flow and regulating the desirable flow restriction. Additionally the manually controlled inflation and deflation of the dilatation balloon does not allow an exact control of the flow rate of the blood and therefore represents no exact defined intervention protocol to prevent or reduce reperfusion injury.

It is an objective of the present invention to provide an improved medical device especially adapted for use in post conditioning treatment.

In order to achieve this objective, the present invention provides a medical device adapted for use in post conditioning treatment comprising:
a balloon catheter functionally connected to
at least one controllable automatically driven pump and
a control unit for controlling the pump depending on at least one physiological parameter and/or catheter-specific parameter and/or in correspondence to a predefined, time-dependent algorithm according to a pre-established post conditioning perfusion protocol.

The balloon catheter of the invention may be a catheter suitable to perform angioplasty-like treatments, comprising at least one dilatation balloon disposed on a flexible tubular catheter body. The dilatation balloon is preferably made from a non-elastomeric thin walled polymer material such as polyvinylchloride, polyethylene, various derivatives thereof or other suitable film-forming material, capable of withstanding pressures of ≥ 100 psi, or ≥ 150 psi, without being damaged and without significant or undue stretch or deformation when inflated into the thin-walled balloon. Suitable materials are generally known to those skilled in the art of angioplasty. The material of the dilatation balloon provides for a stable balloon expansion even under high inflation pressure.

The controllable automatically driven pump in the present invention is functionally connected to the balloon catheter; thereby for instance, providing for an accurate controlled inflation and deflation of the balloon. This allows for an even more exact regulation of a fluid flow dependent on the inflation pressure applied to the balloon and the resulting expansion thereof. Thus, the factors of human inconsistency and error in both the dilatation phase and post conditioning treatment can be reduced or avoided. In addition the fluid flow can be exactly regulated in correspondence to more complex algorithms also comprising exact pre-defined filling states of the balloon, allowing an exact modulation of the fluid flow. In particular, the fluid flow is the blood flow.

With the control unit of the invention the pump can be controlled depending on at least one physiological parameter and/or catheter-specific parameter and/or in correspondence to a predefined, time-dependent algorithm according to a pre-established post conditioning perfusion protocol.

For example, different pre-programmed series of sequential inflation and deflation, so-called intermittency patterns, of the balloon in a targeted extent resulting in full sealing or reduced low pressure blood flow at a time can be stored in the control unit and can thus be chosen by the medical person to fit to the needs of the patient. Like that, a high standard quality of the treatment can be achieved. Thus, the factors of human inconsistency and error in the treatment can be reduced or avoided. Thereby it is possible to provide an improved automated and computer controlled treatment.

The control unit of the invention may comprise a micro processor. The control unit may advantageously be capable of storing data, for example relevant parameters of the performed procedure, especially the intermittency patterns, or for example data of physiological parameters of the treated patient. The parameters can be stored in a directory in one or more parametrization files, which can be selected by the medical practicioner. The parametrization file or files may include all parameters, which can be chosen and/or modified for a suitable automated run of the medical device, in particular the pump functionally connected to the catheter. Therefore, this embodiment of the present invention can give additional information about the quality of the treatment to the medical practitioner and helps to further improve the post conditioning treatment as medical method. Standardization and reproduction of optimized performance with the obtained reliable data of the medical treatment are made possible. Even the performance of different medical practitioners can be compared and optimized clinical studies are conceivable.

The control unit of the invention may also comprise or may be functionally connected to a special software to control the pump. Said software can be parametrized via the parametrization files, in particular with arbirtrary intermittency patterns, for suitable raising or lowering the pressure inside the balloon in order to decrease or increase the fluid flow, especially the reperfusion flow. The automated system of the invention offers increased versatility concerning the intermittency patterns and improved accuracy of the pressure control.

Thus, with the use of an automatically controlled pump and thus automatically controlled catheter according to the invention an angioplasty or angioplasty-like treatment can be improved. Especially reperfusion injury in the post conditioning phase, in the final restoring of the blood flow with the use of post conditioning treatment, can be reduced or even avoided.

In one embodiment of the invention the automatically driven pump is adapted for the pumping, holding and/or suction of a fluid. With the medical device of the invention it is possible to perform repeated and rapid inflation and deflation automatically in either the dilatation phase of angioplasty or reperfusion for final restoring of the blood flow with the automatically driven pump functionally connected to the inflation lumen of the balloon. Therefore the pump can be used to start, to stop and/or to modulate and reduce the blood flow to a certain extend. With the automatically controlled inflation/deflation cycles especially reperfusion can be performed in a secure and smooth manner.

According to one aspect of the invention the pump is drivable at different adjustable pumping rates. Thus, the inflation and deflation rate can be performed in a predetermined pace. For example the blood flow can be started slowly and can be gradually increased until the normal blood flow is achieved.

In one embodiment of the invention the pump is a syringe pump. Furthermore, in a preferred aspect of this embodiment of the medical device of the invention the syringe pump can be a programmable syringe pump.

According to another aspect of the invention the physiological parameter which is chosen to be used in the control of the pump is selected from the group consisting of systemic blood pressure, blood pressure before the balloon catheter, blood pressure behind the balloon catheter, blood flow before the balloon catheter, blood flow behind the balloon catheter, the heart rate and/or electrocardiogram and can advantageously be used to stay within desired physiological limits. The measured physiologic signals, for example monitored by pressure sensors and/or flow sensors integrated in the balloon catheter, or positioned on the balloon catheter, can be transmitted to the control unit which in turn controls the pump and thereby the inflation and deflation of the balloon.

According to another aspect of the invention the catheter-specific parameter is the inflation pressure of the balloon and/or the perfusion pressure of a pumped fluid and/or the volume of the pumped fluid. The balloon inflation and therefore also the degree of obstruction of the blood flow can be controlled, for instance, periodically or continuously based on the pressure signal.

In another embodiment of the present invention the medical device comprises at least one pressure transducer for measuring the physiological parameter and/or catheter-specific parameter and for submitting a signal to the control unit. The transducer is preferably positioned in a sealed fluid connection to the pump. According to the submitted signals the balloon inflation may be controlled, based on the systemic blood pressure or on other above mentioned physiological parameters or catheter specific parameters or a combination of two or more thereof. With the present invention dilatation procedure and the post conditioning treatment with repeated inflation and deflation of the balloon can be performed with continuous or periodic adjustment and/or maintenance of beneficial physiological limits or in beneficial predetermined limits.

According to another aspect of the medical device of the invention the control unit controls the pumping time and/or the pumping volume and thus the filling state and the expansion of the balloon.

According to one embodiment of the present invention the balloon catheter comprises at least two balloons. The first balloon can again be a dilatation balloon disposed on the exterior of the tubular body of the catheter like those used in angioplasty. The second balloon may also be disposed on the exterior of the tubular body. The second balloon can be positioned proximal or distal from the first balloon. It is also possible that a third or multiple balloons are provided. There are also means for inflating and/or deflating the second balloon which can comprise, for instance, a second lumen in the tubular body. The elongate tubular catheter body thus includes a wall which surrounds a plurality of interior lumen. In particular, separate lumens are connected to the interior of the different balloons for separate and independent inflating and deflating of the two balloons. Thus, a first lumen is fluidly connected with the first balloon and a fluid or gas is passed through said first lumen to inflate as well as deflate the first balloon. A second lumen terminates in the second balloon for separate and independent inflation and deflation thereof. The second balloon can act to help in the treatment of a wider area of the occluded vessel. Moreover, it is possible to achieve an exact volume inside the vessel for a restricted and targeted treatment with special drugs, for example rapamycin and/or paclitaxel. Sensor measurements can help to determine the exact pressure of the balloons to just seal the vessel from the blood flow without risking injury of the vessel by over-expansion or by applying too much pressure to the vessel. In addition, the provision of the second balloon can help to create a well defined passage reducing the immediate blood pressure at the first balloon when positioned proximal to the first balloon.

According to another aspect of the invention at least one balloon catheter comprises means for a local delivery of a drug. Thus, the medical device of the present invention can be used for the treatment of occluded or stenotic body tissue or stenotic region in a body lumen, such as an angioplasty procedure alone or in connection with a local drug therapy treatment. Thus a drug can be delivered to a target tissue or body vessel.

Furthermore the means for the local delivery of a drug according to one aspect of the invention comprise a separate lumen in the catheter or a coating on the balloon surface or a delivery via pores in the balloon. The possible local drug delivery can especially be used to treat the occluded segment of a body vessel. Thus, when a combination of an angioplasty procedure and a local drug therapy is necessary or beneficial the medical device of the present invention can reduce the time of the procedure and is cost saving.

The present invention also relates to the use of the medical device of the invention in the treatment of infarctions of organs, for example lung, kidney, liver or splenic infarctions, myocardial infarct or other occlusions of body vessels or body tissues, especially in procedures like angioplasty and/or in therapies based on local drug delivery. In particular, the medical device of the present invention can be advantageously used in post conditioning treatment. With use of the medical device according to one or more before mentioned embodiments of the invention it is possible to reduce or even prevent reperfusion injury or other complication in connection with uncontrolled restoring of the blood flow.

The present invention also relates to the use of the medical device of the invention in the reperfusion of transplanted organs or tissues. Ischemia-reperfusion injuries are also a common complication in organ transplantation, which occurs when blood supply to the transplanted tissues or organs is temporally interrupted and then reintroduced again (reperfusion). With use of the medical device the reperfusion of transplanted organs or tissues can advantageously be controlled and regulated, thereby reducing or even preventing reperfusion injury in the targeted organ or tissue.

The invention will now be described, by way of example only, with reference to the accompanying drawings, in which:
Figure 1 illustrates schematically the components of one embodiment of the medical device according to the invention and
Figure 2 illustrates schematically another embodiment of the medical device according to the invention.

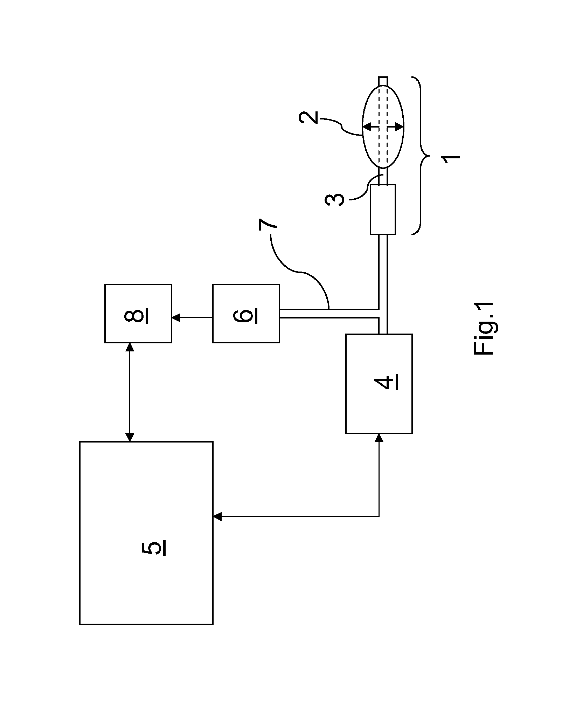

In Figure 1 all components and interconnecting elements between the components are simplified. According to the invention a balloon catheter 1 is equipped with at least one inflatable and deflatable balloon 2. A first lumen 3 is fluidly connected with the balloon 1 and a fluid or gas is passed through said first lumen 3 to inflate as well as deflate the first balloon The catheter comprises a proximal and a distal end and the proximal end of the catheter 1 is fluidly connected to a pump 4. The pump 4 is preferably a programmable syringe pump, which can communicate with and can be controlled by a control unit 5. The control unit 5 may regulate and control the pumping rate, pumping volume and/or the pumping time of the pump 4. A pressure transducer 6 according to the shown embodiment is connected in a sealed fluid connection 7 to the pump 4. The pressure transducer 6 may be connected to an AD converter 8 for data translation and for submitting a signal to the control unit 5. According to the submitted signals the inflation of the balloon 1 may be controlled. The pressure measurements can help to determine the exact pressure of the balloon 2, for example to just seal a body vessel from the blood flow without risking injury of the vessel by over-expansion or by applying too much pressure to the vessel. With the control unit 5 and the programmable syringe pump 4 different pre-programmed series of sequential inflation and deflation of the balloon 2 in a targeted extent resulting in full sealing or reduced low pressure blood flow at a time can be performed and can be chosen by the medical practitioner to fit to the needs of the patient. The balloon inflation and therefore also the degree of obstruction of the blood flow can be controlled periodically or continuously based on the pressure signal. Like that, a high standard quality of the treatment can be achieved. Thus, the factors of human inconsistency and error in the treatment can be reduced or avoided. Thereby it is possible to provide an improved automated and computer controlled treatment.

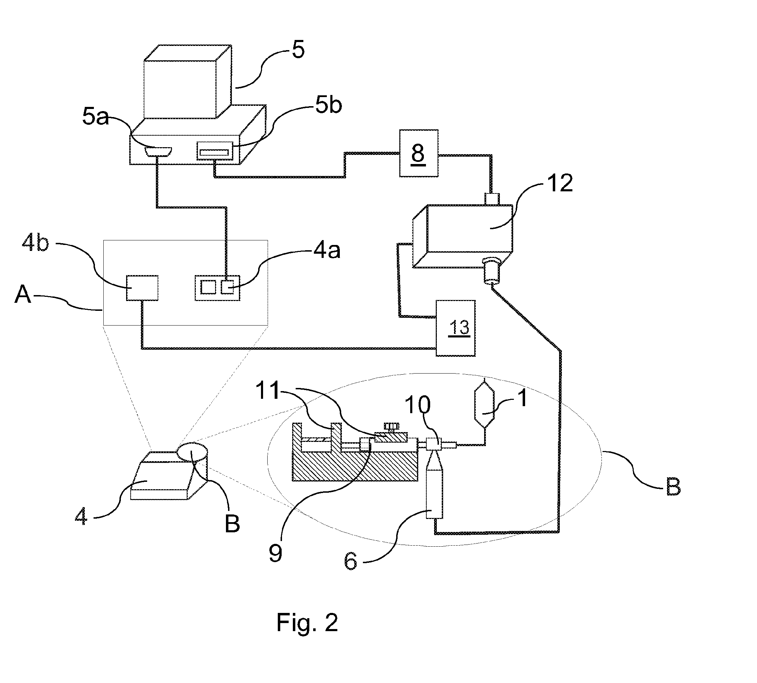

In Figure 2 also all components and interconnecting elements between the components are simplified. The control unit 5 is a PC which is connected via its port 5a with suitable connection cable to the port 4a of the pump 4. Box A illustrates the rear side of the pump 4. The pump 4 may be an infusion pump, for example Harvard Apparatus PHD - 2000. A syringe 9 suitable for use with the pump 4 is connected to a pressure sensor 6 via a connection valve 10. The catheter 1, particularly a dilatation catheter, is connected to the other end of the valve 10. The system may then be filled with the necessary fluids. The syringe 9 is inserted in the infusion pump's 4 syringe fixtures 11. The pressure sensor 6, for example Sensor Technics CTE8015GY0-X, is connected to an adapter device 12, which is connected to an A/D converter 8, for example Data Translations Econ Series DT-9813, on its other end. The pump 4 with its port 4b and the Adapter device are connected to a power supply 13. Other necessary power supplies, for example for the control unit 5, are not shown. The A/D-Converter 8 is connected to the control unit 5 via its port 5b. The connection may be established for example via USB cable. The control unit 5 may comprise software, or may be functionally connected to a software product, for controlling the infusion pump 4. For example the pressure inside the dilatation balloon 2 of the catheter 1 may be controlled by pushing the plunger into the syringe 9 or pulling it out. The software can be parametrized with arbitrary intermittency patterns and raises or lowers the pressure inside the balloon in order to decrease or increase the flow of a fluid, especially the reperfusion flow. The intermittency patterns may correspond to a predefined, time-dependent algorithm according to a pre-established post conditioning perfusion protocol. The software of the invention is adapted to the hardware equipment chosen and to the intended treatment.

The provided medical device with its automated system offers increased versatility and an improved accuracy, especially of the pressure control in reperfusion treatment.

## Claims

1. A medical device adapted for use in post conditioning treatment comprising:
a balloon catheter functionally connected to
at least one controllable automatically driven pump and
a control unit for controlling the pump depending on at least one physiological parameter and/or catheter-specific parameter and/or in correspondence to a predefined, time-dependent algorithm according to a pre-established post conditioning perfusion protocol.

2. The medical device according to claim 1 **characterized in that** the pump is adapted for the pumping, holding and/or suction of a fluid.

3. The medical device according to claim 1 or 2 **characterized in that** the pump is drivable at different adjustable pumping rates.

4. The medical device according to one of the claims 1 to 3 **characterized in that** the pump is a syringe pump.

5. The medical device according to claim 4 **characterized in that** the syringe pump is a programmable syringe pump.

6. The medical device according to according to one of the preceding claims **characterized in that** the physiological parameter is selected from the group consisting of systemic blood pressure, blood pressure before the balloon catheter, blood pressure behind the balloon catheter, blood flow before the balloon catheter, blood flow behind the balloon catheter, heart rate and/or electrocardiogram.

7. The medical device according to one of the preceding claims **characterized in that** the catheter-specific parameter is the inflation pressure of the balloon and/or the perfusion pressure of a pumped fluid and/or the volume of the pumped fluid.

8. The medical device according to one of the preceding claims **characterized in that** the medical device comprises at least one pressure transducer for measuring the physiological parameter and/or catheter-specific parameter and for submitting a signal to the control unit.

9. The medical device according to claim 8 **characterized in that** the transducer is positioned in a sealed fluid connection to the pump.

10. The medical device according to one of the preceding claims **characterized in that** the control unit controls the pumping time and/or the pumping volume.

11. The medical device according to one of the preceding claims **characterized in that** the balloon catheter comprises at least two balloons.

12. The medical device according to one of the preceding claims for use in the treatment of infarctions of organs or body tissues, myocardial infarct, or other occlusions of body vessels or body tissues.

13. The medical device according to one of the preceding claims 1 to 12 for use in the reperfusion of transplanted organs or tissues.
